(19) Europäisches Patentamt
European Patent Office
Office européen des brevets

(11) **EP 4 557 307 A1**

(12) **EUROPEAN PATENT APPLICATION**

(43) Date of publication:
**21.05.2025 Bulletin 2025/21**

(21) Application number: **23210805.0**

(22) Date of filing: **20.11.2023**

(51) International Patent Classification (IPC):
**G16H 50/20** (2018.01)  **G16H 50/30** (2018.01)
**G16H 50/50** (2018.01)  **A61B 5/00** (2006.01)

(52) Cooperative Patent Classification (CPC):
**G16H 50/30; A61B 5/7275;** G16H 50/20;
G16H 50/50

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **KOOIJMAN, Gerben**
**Eindhoven (NL)**
• **JOHNSON, Mark Thomas**
**Eindhoven (NL)**
• **PALERO, Jonathan Alambra**
**Eindhoven (NL)**
• **SPELT, Hanne Adriana Alijda**
**Eindhoven (NL)**
• **ZWARTKRUIS-PELGRIM, Petronella Hendrika**
**Eindhoven (NL)**

(74) Representative: **Philips Intellectual Property & Standards**
**High Tech Campus 52**
**5656 AG Eindhoven (NL)**

(54) **RISK PREDICTION OF PREECLAMPSIA**

(57) Disclosed concepts aim to provide schemes, solutions, concepts, designs, methods, and systems pertaining to improved prediction of a risk of a subject developing preeclampsia. Specifically, a gestational weight gain model is leveraged to predict future gestational weight of a subject based on weight of the subject during at least two previous points in time. Using the predicted gestational weight gain, a preeclampsia prediction model may generate a preeclampsia risk score indicative of a risk of the subject developing preeclampsia. That is, the gestational weight gain of the subject at a future point in time may be used to acquire a more accurate prediction of the risk of the subject developing preeclampsia than with only historical or present weight data. Indeed, preeclampsia models are more accurate when provided with data regarding weight of a subject during a late stage of pregnancy.

FIG. 4

**Description**

FIELD OF THE INVENTION

The present invention relates to the field of preeclampsia risk prediction, and more specifically to prediction of a risk of a subject developing preeclampsia.

BACKGROUND OF THE INVENTION

Hypertensive disorders of pregnancy (HDPs) comprise some of the most common medical complications in pregnancy, affecting up to 10% of all pregnancies. HPDs include gestational hypertension, white-coat hypertension, masked hypertension, chronic hypertension, chronic hypertension with superimposed preeclampsia, preeclampsia, eclampsia, haemolysis, elevated liver enzymes and low platelets (HELLP) syndrome, and postpartum hypertension. These hypertension disorders can contribute to health problems during pregnancy, such as intrauterine growth restriction, placental abruption, preterm birth, and caesarean birth.

Preeclampsia affects 2% to 8% of pregnancies and is a leading cause of maternal morbidity and mortality worldwide. Indeed, there are over 63,000 cases of maternal deaths worldwide attributable to preeclampsia reported annually. The rates of preeclampsia in the United States have been steadily rising being a leading cause of maternal and fetal/neonatal morbidity and mortality.

It is known that having chronic or gestational hypertension increases the risk of developing preeclampsia. Between 13% and 40% of pregnant women with chronic hypertension (present before or developed in the first 20 weeks of pregnancy) will go on to develop superimposed preeclampsia. In those with severe hypertension, the rate of developing superimposed preeclampsia can be as high as 78%. Furthermore, of women initially diagnosed with gestational hypertension (developed after 20 weeks of pregnancy), 15% to 46% will develop preeclampsia. The risk of progression to preeclampsia is inversely related to the gestational age at which gestational hypertension is diagnosed.

Early identification of women at higher risk for preeclampsia could potentially aid early prevention and treatment. Although a plethora of preeclampsia prediction models have been developed in recent years, individualized prediction of preeclampsia is rarely used in clinical practice.

There is therefore a need for improvement of the accuracy of risk prediction of preeclampsia, in particular of the accuracy of risk prediction of preeclampsia in the early stages of pregnancy.

SUMMARY OF THE INVENTION

The invention is defined by the claims.

According to examples in accordance with an aspect of the invention, there is provided a method for predicting a risk of a subject developing preeclampsia.

The method comprises obtaining measured gestational weight data describing a weight of the subject at two or more previous points in time;

processing, with a gestational weight gain prediction model, the measured gestational weight data to generate a predicted gestational weight gain value describing a gestational weight gain of the subject at a future point in time; and processing, with a preeclampsia prediction model, the predicted gestational weight gain value to generate preeclampsia risk score indicative of a risk of the subject developing preeclampsia.

Disclosed concepts aim to provide schemes, solutions, concepts, designs, methods, and systems pertaining to improved prediction of a risk of a subject developing preeclampsia. Specifically, a gestational weight gain model is leveraged to predict future gestational weight of a subject based on weight of the subject during at least two previous points in time. Using the predicted gestational weight gain, a preeclampsia prediction model may generate a preeclampsia risk score indicative of a risk of the subject developing preeclampsia. That is, the gestational weight gain of the subject at a future point in time may be used to acquire a more accurate prediction of the risk of the subject developing preeclampsia than with only historical or present weight data. Indeed, preeclampsia models are more accurate when provided with data regarding weight of a subject during a late stage of pregnancy.

In essence, gestational weight gain at a later stage in pregnancy (e.g., 2nd or 3rd trimester) is predicted using measured gestational weight in an early stage of pregnancy (e.g., first trimester/first 100-140 days). The predicted gestational weight gain is then provided to a preeclampsia prediction model that can determine a risk of developing preeclampsia based on the predicted gestational weight gain. This may lead to generation of a more accurate (i.e. closer to a ground truth) preeclampsia risk score than one that may be generated simply by measured gestational weight data.

Put another way, the invention combines a gestational weight gain prediction model alongside a preeclampsia prediction model to predict (at an early stage) a preeclampsia risk score indicative of a risk of developing preeclampsia.

Typically, measured gestation weight data of the subject is used to determine a risk of developing preeclampsia. Generally, the prediction of the risk will be more accurate with additional and later (i.e. a later gestational age) gestational weight data of the subject. It is therefore proposed to process such measured gestational weight data to predict future gestational weight gain, which may then be used to generate a more accurate preeclampsia risk score.

Once a preeclampsia risk score is generated, medical professionals and/or further algorithms may process the preeclampsia risk score to determine an appropriate course of action for the subject. As a result (with appropriate action taken), there may be improved outcomes for the subject. Indeed, an early and accurate prediction of preeclampsia may enable preventative action to be taken, and for mitigating actions to be taken in a timely manner.

The predicted gestational weight gain value may be a numerical value indicative of the predicted weight of the subject at the future point in time, or a categorical value describing the predicted weight of the subject at the future point in time compared to an ideal weight.

It is thus worth noting that the predicted gestational weight gain value does not need to be a predicted numerical value of the weight gain, but a relative measure indicative of the likely gestational weight gain comparable to an ideal/target gestational weight gain. Indeed, a categorial value (e.g., dangerously low, low, near-target, high, dangerously high) may be sufficient to generate a preeclampsia risk score.

The measured gestational weight data may comprise a weight value describing a weight of the subject at the beginning of pregnancy.

At least one of the one or more previous points in time at which the gestational weight data was measured may correspond to the beginning of the pregnancy of the subject. That is, the measured gestational weight data has at least one data point describing the weight of the subject at the beginning of pregnancy. In some cases, this may be the weight of the subject before pregnancy, which can be used to understand the likely weight of the subject at the beginning of pregnancy. This reflects the weight of the subject before any gestational weight has been gained. Of course, the measured gestational weight data will at least also describe the weight of the subject at another point of time during pregnancy (e.g., at a point during the first trimester).

Furthermore, the measured gestational weight data may comprise weight values describing a weight of the subject at least once per week.

That is, the measured gestational weight data describes the gestational weight of the subject at a point in time at least once per week. By ensuring the gestational weight of the subject is measured at least once per week, a more accurate prediction of the future weight of the subject may be achieved.

In addition, the measured gestational weight data may comprise weight values describing a weight of the subject at the same time of day and/or the same day of the week, each week.

That is, the measured gestational weight data contains multiple different values describing the gestational weight of the subject at the same time of day and/or the same day of the week, on different days/weeks.

Measuring the gestational weight of the subject at the same time of day and/or the same day of week may mean that a trend of the gestational weight of the subject may be more apparent. Indeed, by measuring the gestational weight at the same time of day and/or day of the week may ensure that regular fluctuations in weight throughout the day (e.g., due to eating, exercise, and bowel movements) do not impact the comparability of the gestational weight data between time points. Accordingly, a more accurate prediction of the future weight of the subject may be achieved.

The measured gestational weight data may comprise weight values describing a weight of the subject spanning at least the first 100 days of pregnancy.

That is, the measured gestational weight data contains multiple values which together describe the gestational weight of the subject throughout the first 100 days (and preferably the first 140 days, or the first trimester). This does not necessarily mean that the weight is measured continuously, or even every day or week, but that a trend of the weight of the subject during the first 100 days (at least) is measured.

Accordingly, a more accurate prediction of the future weight of the subject may be achieved.

In some embodiments, the gestational weight gain prediction model may be an extrapolation based prediction model, or a machine-learning based prediction model.

The gestational weight gain model may be implemented using various different prediction algorithms/methods for detecting trends in historical (measured) data to determine a likely future value of the data.

In one case, the gestational weight gain model may be extrapolation based. For example, the gestational weight gain model may apply a maximum a-posteriori regression analysis to the gestational weight data, and extrapolate the data using a fitted function to predict gestational wight gain at a future point in time. In another example, a multinomial logistic regression may be used to predict gestational wight gain at a future point in time. Of course, other extrapolation or regression based methods would be readily applied to the measured gestational weight data.

Furthermore, the gestational weight gain model may be machine-learning based. That is, the gestational weight gain model may be trained by a training algorithm using training data using historic gestational weight data and known future gestational weight gain. As a result, the machine-learning based model may receive measured gestational weight data and output a future gestational weight gain.

The method may further comprise obtaining weight-related physiological data describing at least one physiological parameter of the subject; and adjusting the predicted gestational weight gain value based on the obtained weight-related physiological data.

Of course, a future gestational weight gain may also depend on various physiological parameters of the subject. In other words, physiological parameters may indicate the general/average trend of gestational weight gain taking into account previous (measured gestational weight data. Accordingly, a more accurate prediction of the future weight of the subject may be achieved.

Examples of physiological parameters relevant to gestational weight gain include, the at least one physiological parameter may comprise at least one of a pre-pregnancy weight, a pre-pregnancy BMI, an age, a height, an ethnicity, a stress value, a pre-pregnancy lifestyle parameter value, or a pregnancy lifestyle parameter value.

Indeed, various lifestyle choices may impact the future gestational weight gain value. Such lifestyle choices/parameters may include a level of physical activity, a nutritional intake, a sleep quality/quantity value, and a mental wellbeing metric.

In some embodiments, the method may further comprise obtaining preeclampsia-related physiological data describing at least one physiological parameter of the subject; and adjusting the preeclampsia risk score based on the obtained preeclampsia-related physiological data.

Similarly to future gestational weight gain, a risk of developing preeclampsia may also depend on various physiological parameters of the subject. Indeed, parameters other than gestational weight gain may be relevant to a risk of the subject developing preeclampsia such as an age, a gravidity, a parity, a stress value, a family diabetes record, a historical preeclampsia record, an ethnicity, a stress value, a lifestyle parameter value (e.g., physical activity, nutritional intake, sleep quality and/or quantity, and mental wellbeing value), and historical urine parameters of the subject.

Accordingly, by taking one or more of these parameters into account, a more accurate preeclampsia risk score may be generated.

In further embodiments, the method may further comprise generating a blood pressure measurement recommendation describing a recommended frequency to measure blood pressure of the subject based on the preeclampsia risk score.

The preeclampsia risk score may be used to inform a recommended frequency at which to measure blood pressure of the subject. For example, if the preeclampsia risk score indicates that the subject is likely (more likely than the average) to develop preeclampsia, then the recommended frequency of blood pressure measurements may be increased in order to detect issues and take preventive and/or mitigating actions as soon as possible.

In additional embodiments, the method may further comprise generating a lifestyle recommendation describing a recommended change to a lifestyle of the subject to reduce a risk of preeclampsia based on the preeclampsia risk score.

The preeclampsia risk score may be used to inform lifestyle choices of the subject. For example, if the preeclampsia risk score indicates that the subject is likely (more likely than the average) to develop preeclampsia, then recommendations may be generated for reducing the chance of developing preeclampsia, therefore providing early intervention Specifically, nutritional or exercise routine changes may be automatically generated and recommended.

In yet another embodiment, the method may further comprise generating a medical intervention recommendation describing a recommended treatment or diagnostic procedure for the subject based on the preeclampsia risk score.

Indeed, the preeclampsia risk score may be used to inform various medical interventions, procedures or diagnostic actions. For example, if the preeclampsia risk score indicates that the subject is likely (more likely than the average) to develop preeclampsia, then the subject may be prompted to visit a health care provider for initial screening for gestational diabetes.

In some embodiments, the method may also comprise obtaining a pregnancy risk score indicative of a risk of complications in pregnancy of the subject; and modifying the pregnancy risk score based on the preeclampsia risk score.

An overall pregnancy risk score may be updated responsive to the generation of the preeclampsia risk score. This updated pregnancy risk score may therefore better represent a ground truth of the risk of complications occurring during pregnancy of the subject. An improved (i.e. more effective and efficient) allocation of time and resources may be achieved by virtue of the more accurate pregnancy risk score.

According to other examples in accordance with a further aspect of the invention, there is provided a computer program comprising computer program code means adapted, when said computer program is run on a computer, to implement the method of determining vascular compliance of a subject and/or determining hemodynamic parameters of a subject.

According to further examples in accordance with the invention, there is provided a system for predicting a risk of a subject developing preeclampsia.

The system comprises an interface configured to obtain measured gestational weight data describing a weight of the subject at two or more previous points in time; and
a processor configured to:

process, with a gestational weight gain prediction model, the measured gestational weight data to generate a predicted gestational weight gain value describing a gestational weight gain of the subject at a future point in time; and process, with a preeclampsia prediction model, the predicted gestational weight gain to generate preeclampsia risk score indicative of a risk of the subject developing preeclampsia.

In some embodiments, the system may further comprise a weight measurement unit configured to measure the gestational weight data.

The weight measurement unit may be a set of scales that automatically measure and store the weight of the subject for processing to determine the preeclampsia risk score. Accordingly, minimal to no user input may be required (other than the subject being present for weighing, which may otherwise usually form part of their routine).

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 presents a flow diagram of a method for predicting a risk of a subject developing preeclampsia according to an embodiment of the invention;
Fig. 2 presents a flow diagram of a method for using the predicted preeclampsia risk score according to another aspect of the invention;
Fig. 3 presents a block diagram of a system for predicting a risk of a subject developing preeclampsia according to a further aspect of the invention; and
Fig. 4 is a simplified block diagram of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Proposed concepts provide an improved means for predicting a risk of a subject developing preeclampsia. Specifically, a gestational weight gain model is leveraged to predict future gestational weight of a subject based on weight of the subject during at least two previous points in time. Using the predicted gestational weight gain, a preeclampsia prediction model may generate a preeclampsia risk score indicative of a risk of the subject developing preeclampsia. That is, the gestational weight gain of the subject at a future point in time may be used to acquire a more accurate prediction of the risk of the subject developing preeclampsia than with only historical or present weight data. Indeed, preeclampsia models are more accurate when provided with data regarding weight of a subject during a late stage of pregnancy.

It has been observed that the risk prediction accuracy of preeclampsia models increases when provided with gestational weight of the subject during the 2nd and 3rd trimester (i.e. later stages of pregnancy). However, providing a preeclampsia model with such data necessarily requires the subject to be in a later stage of pregnancy for the data to have been measured. At this later stage, prediction of preeclampsia is of limited utility, as it is already too late in the pregnancy to perform many preventative or mitigating acts.

It is therefore an object of the present invention to provide methods for providing accurate and early risk prediction of preeclampsia based on measured weight earlier in the pregnancy, such as within the first trimester. That is, the invention aims to improve the accuracy of models that predict preeclampsia, whilst still enabling such predictions to be performed at an early stage of pregnancy when preventative or mitigating action against preeclampsia may be taken.

Generally, exemplary embodiments of the present invention may comprise the following steps:

(i) measuring and recording gestational weight of the subject. The gestational weight will preferably be measured at least once a week, and preferably at the same time of day (e.g., at 10am every day) and during the same day of the week (e.g., every Monday). The gestational weight will preferably be measured until a gestational age of 100 days, or until a gestational age of 140 days;

(ii) predicting the gestational weight gain value at one or more later time points (e.g., in the 2nd and/or 3rd trimester) based on the measured gestational weight data. The gestational weight gain value may be either numerical (e.g., a weight value) or categorical (e.g., classification based on IOM 2009 guidelines);

(iii) predicting risk or risk-level of the subject developing preeclampsia (i.e. a preeclampsia risk score) by providing the predicted gestational weight gain value(s) to a trained preeclampsia prediction model; and

(iv) optionally, the preeclampsia risk score is communicated to a user (the subject or a medical professional). The preeclampsia risk score may also be used to generate and/or modify guidance or recommendations for preventing or mitigating preeclampsia, which may also be communicated to a user. For example, the recommendation may be to measure and record blood pressure data more frequently.

Each of the above steps may be repeated continuously as additional gestational weight measurements of the subject are obtained. That is, the preeclampsia risk score may be continually updated responsive to new/additional gestational weight data becoming available. Accordingly, a dynamic risk or risk-level prediction of preeclampsia may be acquired, accounting for recent changes in the weight of the subject. This may be particularly relevant should the subject make lifestyle changes (e.g., nutrition or exercise changes) during pregnancy that may alter their longer term gestational weight gain.

In essence, embodiments of the invention are based on the fact predicting future gestational weight gain from two or more previous weight measurements. Then, from the gestational weight gain prediction, a preeclampsia risk level can be predicted.

Advantageously, the invention means that there is not a need for an annotated dataset containing both weight measurements and an associated preeclampsia diagnosis. Instead, the invention couples two separate models - one for gestational weight gain and one from preeclampsia - that can be trained based on separate datasets (e.g., one having historic gestational weight values and known future gestational weight gain, and another having gestational weight gain values and known preeclampsia diagnosis.

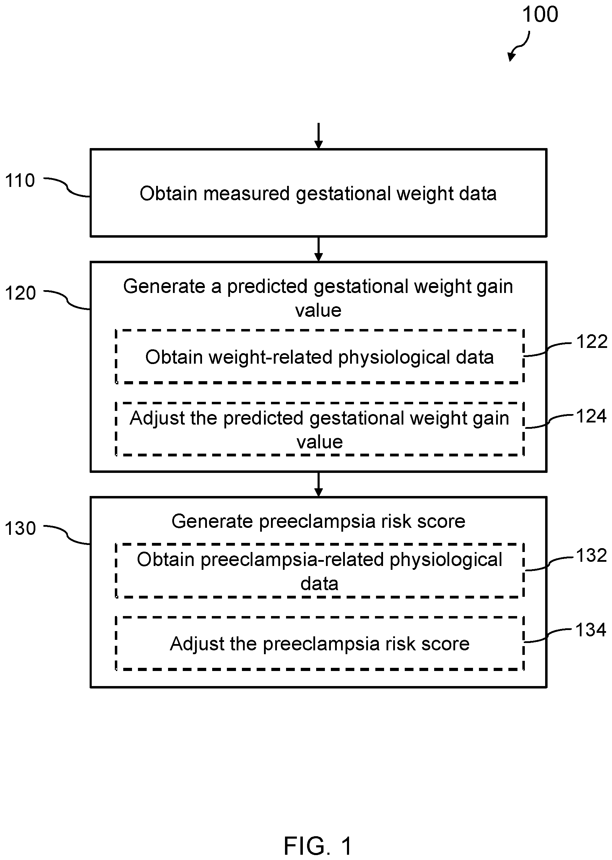

Turning to Fig. 1, there is presented a flow diagram of a method for predicting a risk of a subject developing preeclampsia. In some embodiments, the method may be used to accurately predict preeclampsia during an early stage of pregnancy (i.e. during/at the end of the first trimester).

In step 110, measured gestational weight data is obtained. The measured gestational weight data describes the weight of the subject at two or more previous points in time. That is, the measured gestational weight data comprises weight values corresponding to at least two previous points in time during (the same) pregnancy of the subject.

The measured gestational weight data may be obtained from storage (e.g., electronic medical records, or a personal measuring device/scales), or acquired/measured directly for use in the method.

Preferably, the measured gestational weight data may comprise weight values describing a weight of the subject spanning at least the first 100 days of pregnancy, and preferably the first 140 days of pregnancy. By acquiring weight data describing (at least a trend of) weight of the subject over this time period, an accurate prediction of the gestational weight gain of the subject over the remainder of the pregnancy may be acquired. Indeed, this is still early enough in the pregnancy that preventative and/or mitigating actions can be taken regarding the potential for preeclampsia.

Nevertheless, the gestational weight gain of the subject may be predicted (although not as accurately) using only two gestational weight measurements. Of course, preferably there will be further gestational weight measurements in order to improve accuracy of the predicted future gestational weight gain.

In some cases, the measured gestational weight data comprises a weight value describing a weight of the subject at a beginning of pregnancy. This may be a weight value taken before the pregnancy (and therefore indicative of the weight at the beginning of the pregnancy).

Optionally, the measured gestational weight data may comprise weight values describing a weight of the subject at least once per week. With increasing frequency of the gestational weight of the subject, a clearer picture of the historical gestational weight gain can be gathered. Furthermore, the measured gestational weight data may comprise weight values describing a weight of the subject at the same time of day and/or the same day of the week, each week.

In step 120, the measured gestational weight data is processed with a gestational weight gain prediction model. Accordingly, in step 120, a predicted gestational weight gain value is generated, which describes a gestational weight gain of the subject at a future point in time. The gestational weight gain of the subject at a future point in time may correspond to a possible/likely gestational weight gain of the subject at a point later on in pregnancy (e.g., in the second or third trimester).

The predicted gestational weight gain value may be a numerical value indicative of the predicted weight of the subject at the future point in time. That is, the predicted gestational weight gain value may be a quantitively-defined value describing a weight of the subject at the later point in time.

Alternatively, the predicted gestational weight gain value may be a categorical value describing the predicted weight of the subject at the future point in time compared to an ideal weight. The categorical value may be, for example, a description of the gestational weight gain being high, low, or average compared to an expected/ideal/target gestational weight gain. Such a categorisation may be based on IOM 2009 guidelines.

More specifically, the gestational weight gain prediction model may be an extrapolation/regression based prediction model, or a machine-learning based prediction model. Indeed, any method of predicting future gestational weight based on measured (previous) gestational weight of the subject may be utilised by the invention.

By way of illustrative example, the gestational weight gain prediction model may comprise the following steps:

(i) applying maximum a-posteriori (MAP) regression analysis to the measured gestational weight data;
(ii) extrapolating the data using the fitted function of the MAP regression analysis to predict a gestational weight gain value corresponding to later time points (e.g., second and/or third trimester).

In another exemplary embodiment, the gestational weight gain prediction model may further comprise the following steps:

(i) applying a multinomial logistic regression (MLR) method to the following:

a. the predicted gestational weight gain classification obtained by the MAP regression method;
b. scaled difference between measured weight gain and IOM guidelines at each available measurement as:

$$scaled\ weight\ gain\ difference\ with\ IOM = \frac{weight\ gain - 0.5(IOM_{upper} + IOM_{lower})}{0.5(IOM_{upper} - IOM_{lower})}$$

c. metadata including one or more of pre-pregnancy weight, pre-pregnancy BMI or height, age, ethnicity, pre-pregnancy lifestyle parameters (tracked or self-reported physical activity levels, sleep, nutrition, mental-wellbeing), and pregnancy lifestyle parameters (tracked or self-reported physical activity levels, sleep, nutrition, mental-wellbeing).

(ii) predict categorical gestational weight gain value (e.g., classification based on IOM 2009 guidelines) corresponding to later time points.

For the classifier, alternative methods include support vector machine (SVM) and random forest (RF).

In yet another embodiment, the gestational weight gain prediction model may further comprise the following steps:

(i) selecting a subset from the gestational weight data over preferably the full pregnancy period. The subset selection is based on similarity in gestational weight gain data over the available corresponding timeframes (and possibly additional parameters);
(ii) applying a regression method, such as a (3rd) order polynomial function, MAP or Gaussian Processes Regression (GPR), using the selected subset;
(iii) extrapolate the measured gestational weight data using the fitted function to predict gestational weight gain at later time points.

Moreover, in some embodiments, the predicted gestational weight gain value may be further based on weight-related physiological parameters. This consideration may be integrated with the gestational weight gain prediction model, or may be applied after the gestational weight gain prediction model provides a predicted gestational weight gain value.

Specifically, Step 120 may also comprise further additional sub-steps 122 and 124.

In sub-step 122, weight-related physiological data is obtained. Said data describes at least one physiological parameter of the subject that relates to gestational weight gain. This may be obtained from a database (e.g., electronic medical records), or may be self-reported information obtained directly for the purpose of updating the predicted gestational weight gain value.

The at least one physiological parameter comprises at least one of a pre-pregnancy weight, a pre-pregnancy BMI, an age, a height, an ethnicity, a stress value, a pre-pregnancy lifestyle parameter value, or a pregnancy lifestyle parameter value. Each of these physiological parameters may indicate the trend/pattern of gestational weight gain of the subject. Of course, further weight-related physiological parameters may be utilised, and would be readily apparent and identified by the skilled person.

In sub-step 124, the predicted gestational weight gain value is adjusted based on the obtained weight-related physiological data.

In step 130, the predicted gestational weight gain value is processed with a preeclampsia prediction model. As a result, a preeclampsia risk score indicative of a risk of the subject developing preeclampsia is generated.

The preeclampsia prediction model may be any known model for predicting the risk of a subject developing preeclampsia from weight data of a subject, as would be readily appreciated by the skilled person. In this case, the (predicted) gestational weight gain value is processed to generate the preeclampsia risk score. Of course, the gestational weight gain value may be pre-processed to be in the format required by the preeclampsia prediction model (e.g., an absolute weight value, a relative weight value compared to a target weight value, or a categorical weight value).

Nevertheless, the measured (i.e. previous/historical) weight data of the subject may also be input to the risk prediction model, along with the predicted gestational weight gain value.

Furthermore, preeclampsia-related physiological data may also be input to the preeclampsia prediction model to account for physiological differences in the subject that may be indicative of a susceptibility to preeclampsia. Alternatively, the preeclampsia risk score may be adjusted after generation based on preeclampsia-related physiological data.

Indeed, in (optional) sub-step 132, preeclampsia-related physiological data describing at least one physiological parameter of the subject is obtained. This may be obtained from a database (e.g., electronic medical records), or may be self-reported information obtained directly for the purpose of updating the preeclampsia risk score.

The at least one physiological parameter of the subject may comprise at least one of an age, a gravidity, a parity, a stress value, a family diabetes record, a historical preeclampsia record, an ethnicity, a stress value, a lifestyle parameter value. and historical urine parameters. Of course, further preeclampsia-related physiological parameters may be utilised, and would be readily apparent and identified by the skilled person.

In (optional) sub step 134, the preeclampsia risk score is adjusted based on the obtained preeclampsia-related physiological data. For example, if a family history of preeclampsia indicates a susceptibility to preeclampsia, then the preeclampsia risk score may be adjusted to indicate a greater likelihood of the subject developing preeclampsia.

Steps 110-130 may be repeatedly performed as additional/new measured gestational weight data is obtained, thus adjusting and updating the preeclampsia risk score based on the latest obtained information from the subject.

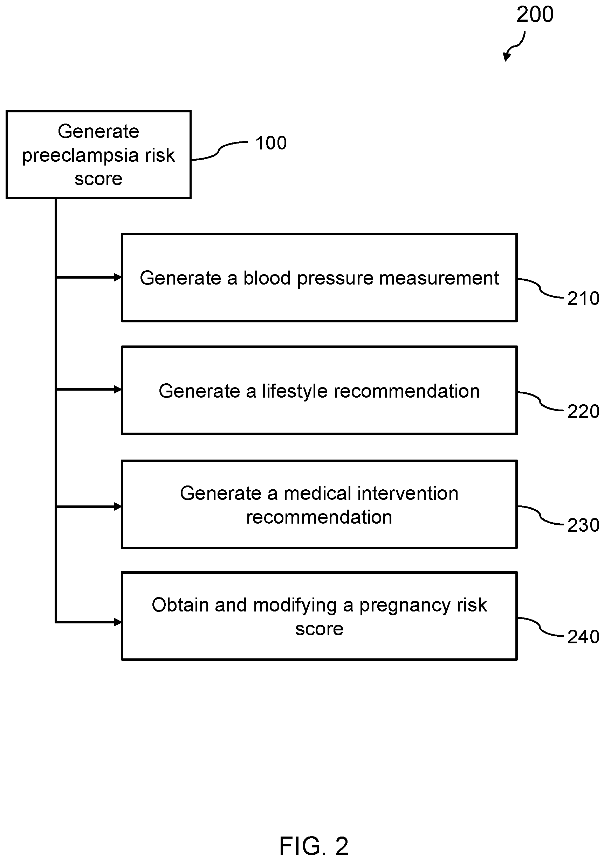

Fig. 2 presents a method for using the predicted preeclampsia risk score according to another aspect of the invention. In step 100, the preeclampsia risk score indicative of a risk of the subject developing preeclampsia is generated according to any method described in relation to Fig. 1. One or more of steps 210-240 may be performed.

In step 210, a blood pressure measurement recommendation is generated based on the preeclampsia risk score. The blood pressure measurement recommendation describes a recommended frequency to measure blood pressure of the subject. That is, if the preeclampsia risk score indicates that the subject is susceptible to preeclampsia, it may be recommended to increase the frequency of blood pressure measurements.

In step 220, a lifestyle recommendation is generated based on the preeclampsia risk score. The lifestyle recommendation describes a recommended change to a lifestyle of the subject to reduce a risk of preeclampsia. Such recommendations may thus provide early intervention and therefore prevent preeclampsia developing. For example, if the preeclampsia risk score indicates a high likelihood of developing preeclampsia, then a strict nutritional and/or exercise regime may be recommended. Furthermore, the lifestyle recommendation may provide personalized recommendations for behaviour change or seeking support (e.g., nutritionist, physical therapist, etc.).

In step 230, a medical intervention recommendation is generated based on the preeclampsia risk score. The medical intervention recommendation describes a recommended treatment or diagnostic procedure for the subject based on the preeclampsia risk score. The medical intervention recommendation may thus provide guidance for early medical intervention (e.g., by providing recommendations to visit a health care provider for initial screening for gestational diabetes).

In step 240, a pregnancy risk score indicative of a risk of complications in pregnancy of the subject is obtained. Said pregnancy risk score is then modified based on the preeclampsia risk score. The pregnancy risk score may be obtained from a pregnancy risk management system, and a risk level is identified and updated based on the preeclampsia risk score.

The outputs of any of steps 210-240 may be output to a user (e.g., the subject or a medical professional), or may be stored for future reference.

It is worth noting that steps 210-240 may not be performed immediately after generating the preeclampsia risk score. That is, the preeclampsia risk score may be stored for a time before being used to perform any of steps 210-240. Furthermore, steps 210-240 may be repeatedly performed as a preeclampsia risk score is updated (e.g., responsive to additional measured gestational weight data being obtained).

In additional, further uses of the preeclampsia risk score would be readily apparent to the skilled person. Indeed, the preeclampsia risk score may simply be presented to the subject and/or a medical profession, such that they use their own judgement for further actions to be taken.

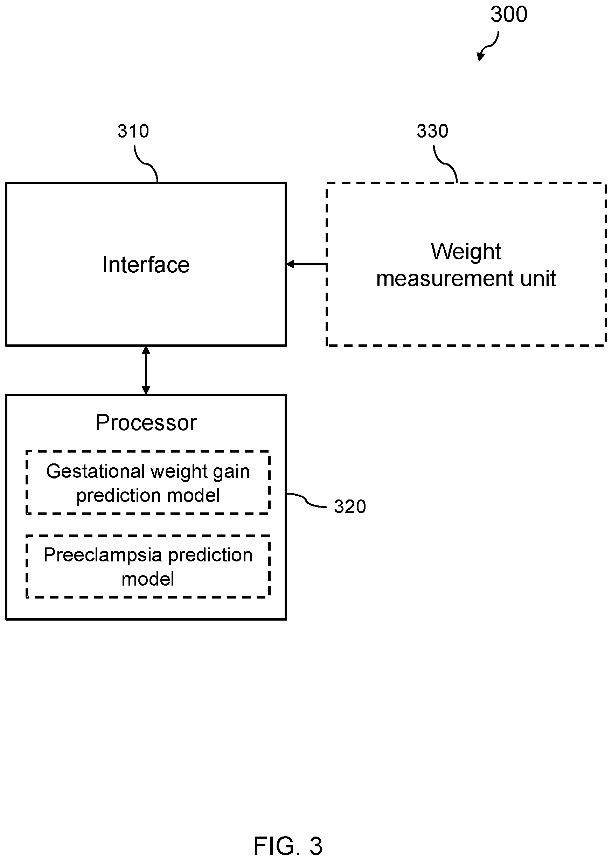

Fig. 3 presents a block diagram of a system 300 for predicting a risk of a subject developing preeclampsia. The system 300 comprises an interface 310, and a processor 320. Optionally, the system 300 further comprises a weight measurement unit 330.

The interface 310 and the processor 320 may be implemented on the same device. That is, the interface 310 and the processor 320 may, or may not, be separate components. Furthermore, if the weight measurement unit 330 is provided, the interface 310 and/or processor 320 may also be integrated with the weight measurement unit 330, or may be provided as a separate device. For example, the interface 310 and processor 320 may be implemented as part for a personal device of the subject (e.g., a smartphone or laptop), or as component(s) of the weight measurement unit 330. Equally the interface 310 and processor 320 may be implemented on the cloud or other remote processing means.

In any case, the interface 310 is configured to obtain measured gestational weight data describing a weight of the subject at two or more previous points in time. The measured gestational weight data may comprise values/describe the weight of the subject as described in reference to Fig. 1.

When the weight measurement unit 330 is provided, the interface 310 may be configured to obtain the measured gestational weight data from the weight measurement unit 330. Of course, the weight measurement unit 330 is configured to measure the gestational weight data. The interface 310 may obtain this data via a wired or wireless connection, or indirectly via a database or other storage.

The processor 320 has access to a gestational weight gain prediction model and a preeclampsia prediction model. In this way, the processor 320 is configured to process, with the gestational weight gain prediction model, the measured gestational weight data to generate a predicted gestational weight gain value describing a gestational weight gain of the subject at a future point in time. Furthermore, the processor 320 is configured to process, with the preeclampsia prediction model, the predicted gestational weight gain value to generate preeclampsia risk score indicative of a risk of the subject developing preeclampsia.

Of course, the processor 320 may perform any of the processes described in relation to step 130 of Fig. 1. Moreover, the processor 320 may further perform any of the steps described in relation to Fig. 2 for using the preeclampsia risk score.

The interface 310 may be further configured to output the preeclampsia risk score, and/or any recommendations generated by the processor 320. Alternatively or additionally, the preeclampsia risk score and recommendations may be stored for later use.

Finally, the system 300 may further comprise other physiological measurement units (e.g., a (connected) blood pressure measurement unit) for generating weight or preeclampsia related physiological parameter values.

Overall, the system 300 of Fig. 3 provides a means for early (and accurate) prediction of preeclampsia so that preventative or mitigating actions may be taken.

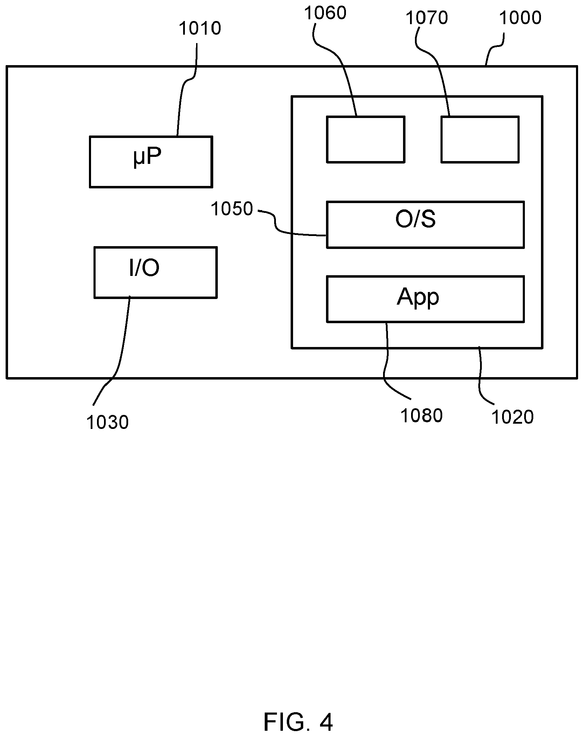

Fig. 4 illustrates an example of a computer 1000 within which one or more parts of an embodiment may be employed. Various operations discussed above may utilize the capabilities of the computer 1000. For example, one or more parts of a system for acquiring an input from a user to control an interface may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, smart phones, PCs, workstations, laptops, PDAs, palm devices, servers, storages, patient monitors and the like. In many cases, the disclosed systems and methods may be implemented in a patient monitor, such as a bed side monitor. Such monitors may be used in, for example, the intensive care unit (ICU), the operating room (OR), or postanaesthesia care unit (PACU).

Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), solid state drive (SSD), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, functional programming and the like.

The I/O devices 1030 may include input devices such as, for example but not limited to, a mouse, keyboard, scanner, microphone, camera, touch-screen, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Figs. 1 and 2, and the system described in relation to Fig. 3, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, EEPROM, and SSD, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram of Fig. 3 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A computer-implemented method (100) for predicting a risk of a subject developing preeclampsia, comprising:

   obtaining (110) measured gestational weight data describing a weight of the subject at two or more previous points in time;
   processing (120), with a gestational weight gain prediction model, the measured gestational weight data to generate a predicted gestational weight gain value describing a gestational weight gain of the subject at a future point in time; and
   processing (130), with a preeclampsia prediction model, the predicted gestational weight gain value to generate preeclampsia risk score indicative of a risk of the subject developing preeclampsia.

2. The method of claim 1, wherein the measured gestational weight data comprises a weight value describing a weight of the subject at a beginning of pregnancy.

3. The method of claim 1 or 2, wherein the measured gestational weight data comprises weight values describing a weight of the subject at least once per week, and optionally wherein the measured gestational weight data describes a weight of the subject at the same time of day and/or the same day of the week, each week.

4. The method of any of claims 1-3, wherein the measured gestational weight data comprises weight values describing a weight of the subject spanning at least the first 100 days of pregnancy.

5. The method of any of claims 1-4, wherein the gestational weight gain prediction model is an extrapolation based prediction model, or a machine-learning based prediction model.

6. The method of any of claims 1-5, further comprising:

   obtaining (122) weight-related physiological data describing at least one physiological parameter of the subject; and

adjusting (124) the predicted gestational weight gain value based on the obtained weight-related physiological data, and
optionally wherein the at least one physiological parameter comprises at least one of a pre-pregnancy weight, a pre-pregnancy BMI, an age, a height, an ethnicity, a stress value a pre-pregnancy lifestyle parameter value, or a pregnancy lifestyle parameter value.

7. The method of any of claims 1-6, wherein the predicted gestational weight gain value is a numerical value indicative of the predicted weight of the subject at the future point in time, or a categorical value describing the predicted weight of the subject at the future point in time compared to an ideal weight.

8. The method of any of claims 1-7, further comprising:

obtaining (132) preeclampsia-related physiological data describing at least one physiological parameter of the subject; and
adjusting (134) the preeclampsia risk score based on the obtained preeclampsia-related physiological data, and optionally wherein the at least one physiological parameter of the subject comprises at least one of an age, a gravidity, a parity, a stress value, a family diabetes record, a historical preeclampsia record, an ethnicity, a stress value, a lifestyle parameter value, and historical urine parameters.

9. The method of any of claims 1-8, further comprising generating (210) a blood pressure measurement recommendation describing a recommended frequency to measure blood pressure of the subject based on the preeclampsia risk score.

10. The method of any of claims 1-9, further comprising generating (220) a lifestyle recommendation describing a recommended change to a lifestyle of the subject to reduce a risk of preeclampsia based on the preeclampsia risk score.

11. The method of any of claims 1-10, further comprising generating (230) a medical intervention recommendation describing a recommended treatment or diagnostic procedure for the subject based on the preeclampsia risk score.

12. The method of any of claims 1-11, further comprising:

obtaining a pregnancy risk score indicative of a risk of complications in pregnancy of the subject;
modifying (240) the pregnancy risk score based on the preeclampsia risk score.

13. A computer program comprising computer program code means adapted, when said computer program is run on a computer, to implement the method of any of claims 1-12.

14. A system (300) for predicting a risk of a subject developing preeclampsia, comprising:

an interface (310) configured to obtain measured gestational weight data describing a weight of the subject at two or more previous points in time;
a processor (320) configured to:

process, with a gestational weight gain prediction model, the measured gestational weight data to generate a predicted gestational weight gain value describing a gestational weight gain of the subject at a future point in time; and
process, with a preeclampsia prediction model, the predicted gestational weight gain value to generate preeclampsia risk score indicative of a risk of the subject developing preeclampsia.

15. The system of claim 14, further comprising a weight measurement unit (330) configured to measure the gestational weight data.

100

110 — Obtain measured gestational weight data

120 — Generate a predicted gestational weight gain value

Obtain weight-related physiological data — 122

Adjust the predicted gestational weight gain value — 124

130 — Generate preeclampsia risk score

Obtain preeclampsia-related physiological data — 132

Adjust the preeclampsia risk score — 134

FIG. 1

200

Generate
preeclampsia risk
score — 100

Generate a blood pressure measurement — 210

Generate a lifestyle recommendation — 220

Generate a medical intervention
recommendation — 230

Obtain and modifying a pregnancy risk
score — 240

FIG. 2

300

310                                              330

Interface          ◄——          Weight
                                 measurement unit

↕

Processor

Gestational weight gain
prediction model                 320

Preeclampsia prediction
model

FIG. 3

FIG. 4

Europäisches
Patentamt

European
Patent Office

Office européen
des brevets

## EUROPEAN SEARCH REPORT

Application Number

EP 23 21 0805

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | OVERCASH RACHAEL T ET AL: "Early Second Trimester Weight Gain in Obese Women Predicts Excessive Gestational Weight Gain in Pregnancy", MATERNAL AND CHILD HEALTH JOURNAL, SPRINGER US, BOSTON, vol. 19, no. 11, 19 June 2015 (2015-06-19), pages 2412-2418, XP035553339, ISSN: 1092-7875, DOI: 10.1007/S10995-015-1760-8 [retrieved on 2015-06-19] | 1,2,4, 6-14 | INV. G16H50/20 G16H50/30 G16H50/50 A61B5/00 |
| Y | * p.2412 col.2 l.2, p.2413 col.1 l.1-5, p.2412 col.1 par.2, p.2413 col.1 par.3, p.2413 col.2 l.7, p.2413 col.2 par.2, p.2415 col.2 l.1, p.2416 col.2 par.2, p.2417 col.2 par.2, p.2412 col.2 par.3 * | 3,5,15 | |
| Y | PURI CHETANYA ET AL: "Gestational weight gain prediction using privacy preserving federated learning", 2021 43RD ANNUAL INTERNATIONAL CONFERENCE OF THE IEEE ENGINEERING IN MEDICINE & BIOLOGY SOCIETY (EMBC), IEEE, 1 November 2021 (2021-11-01), pages 2170-2174, XP034042974, DOI: 10.1109/EMBC46164.2021.9630505 [retrieved on 2021-11-29] | 3,5,15 | |
| A | * abstract, p.2171 col.1 par.2, p.2172 col.1 l.15 * | 1,2,4, 6-14 | TECHNICAL FIELDS SEARCHED (IPC) G16H A61B |
| A | US 2023/132131 A1 (TUYTTEN ROBIN [IE]) 27 April 2023 (2023-04-27) * abstract * | 1-15 | |

The present search report has been drawn up for all claims

| Place of search | Date of completion of the search | Examiner |
|---|---|---|
| Munich | 25 March 2024 | Sundqvist, Benjamin |

EPO FORM 1503 03.82 (P04C01)

## ANNEX TO THE EUROPEAN SEARCH REPORT
## ON EUROPEAN PATENT APPLICATION NO.

**EP 23 21 0805**

This annex lists the patent family members relating to the patent documents cited in the above-mentioned European search report.
The members are as contained in the European Patent Office EDP file on
The European Patent Office is in no way liable for these particulars which are merely given for the purpose of information.

**25-03-2024**

| Patent document cited in search report | | Publication date | Patent family member(s) | | Publication date |
|---|---|---|---|---|---|
| US 2023132131 | A1 | 27-04-2023 | AU | 2021225366 A1 | 06-10-2022 |
| | | | CA | 3169905 A1 | 02-09-2021 |
| | | | CN | 115715165 A | 24-02-2023 |
| | | | EP | 4110164 A1 | 04-01-2023 |
| | | | US | 2023132131 A1 | 27-04-2023 |
| | | | WO | 2021170885 A1 | 02-09-2021 |

EPO FORM P0459

For more details about this annex : see Official Journal of the European Patent Office, No. 12/82